**Europäisches Patentamt**

**European Patent Office**

**Office européen des brevets**

(19)

(11) Publication number : **0 458 378 B1**

(12)

# EUROPEAN PATENT SPECIFICATION

(45) Date of publication of patent specification :
05.01.94 Bulletin 94/01

(51) Int. Cl.$^5$ : **B01J 29/22**, B01J 29/06, C07C 5/27

(21) Application number : **91201063.4**

(22) Date of filing : **03.05.91**

(54) **Catalyst composition.**

The file contains technical information
submitted after the application was filed and
not included in this specification

(30) Priority : **22.05.90 GB 9011412**

(43) Date of publication of application :
27.11.91 Bulletin 91/48

(45) Publication of the grant of the patent :
05.01.94 Bulletin 94/01

(84) Designated Contracting States :
**BE DE FR GB NL**

(56) References cited :
FR-A- 2 211 423
FR-A- 2 477 903
US-A- 3 767 721

(73) Proprietor : **SHELL INTERNATIONALE
RESEARCH MAATSCHAPPIJ B.V.
Carel van Bylandtlaan 30
NL-2596 HR Den Haag (NL)**

(72) Inventor : **Terlouw, Teunis
Badhuisweg 3
NL-1031 CM Amsterdam (NL)**
Inventor : **Gilson, Jean-Pierre
Badhuisweg 3
NL-1031 CM Amsterdam (NL)**

## Description

The invention relates to a catalyst composition, suitable for use in a process for the isomerization of $C_8$-alkyl aromatics, to a process for the preparation of said catalyst composition and to a process for the isomerization of $C_8$-alkyl aromatics.

The major source of $C_8$-alkyl aromatics is petroleum based and comes from reformate (catalytically reformed naphtha) and pyrolysis gasoline (co-produced with ethylene from cracked naphtha/gas oil) distillation. The product stream of these $C_8$-alkyl aromatics is generally richer in ethylbenzene (10-40%) than the thermodynamic equilibrium : m-xylene 47.5%, p-xylene 23%, o-xylene 22.5%, ethylbenzene 7% (at 400 °C). There exists an imbalance between the supply and demand of the various $C_8$-alkyl aromatics; their isomerization is needed so that larger quantities of ortho-xylene and especially the para-xylene are produced.

It is now well established that xylenes isomerization is an acid catalyzed reaction while ethylbenzene isomerization in mixed xylenes is a typical bi-functional reaction requiring both an acid and a hydrogenation/dehydrogenation function and operating under $H_2$ pressure. The most preferred solid acids are the zeolites and the preferred hydrogenation/dehydrogenation compounds are the Group VIII elements (Pt being the most preferred). Next to an isomerization unit is built a separation section where ortho-xylene is recovered by distillation and para-xylene is recovered by crystallization or by selective adsorption on dedicated molecular sieves. The para-xylene and ortho-xylene poor raffinate is sent to the catalytic isomerization unit where the various $C_8$-alkyl aromatics are again produced in equilibrium concentrations.

Under the reaction conditions, unwanted reactions take place, such as disproportionation, dealkylation, coke deposition, which give rise to a loss of $C_8$-alkyl aromatics.

Commercial catalysts, suitable for the isomerization of $C_8$-alkyl aromatics comprise a Group VIII metal, an alkali metal containing mordenite and alumina as binder.

It has been found that the loss of $C_8$-alkyl aromatics is strongly dependent on the sodium content in the mordenite. Generally the loss of $C_8$-alkyl aromatics is high during the isomerization reaction. It has further been found that H-mordenite or mordenite with a low sodium content gives high losses of $C_8$-alkyl aromatics and moreover that mordenite with a high sodium content gives low ATE values for ethylbenzene (EB). The ATE value is a measure for the conversion of ethylbenzene. Knowing the concentration of ethylbenzene in the $C_8$-alkyl aromatics mixture in the feedstock and the product, ATE, approach to equilibrium in per cent is calculated as follows:

$$ATE = \frac{\text{concentration EB in feed} - \text{concentration EB in product}}{\text{concentration EB in feed} - \text{concentration EB equilibrium}} \times 100$$

The equilibrium concentration for ethylbenzene and also for the o-, m- or p-xylene is a constant value at a fixed temperature, determined by thermodynamics.

A high ATE for ethylbenzene is proportional to a low concentration of ethylbenzene in the product, which means that the ethylbenzene has been converted.

Xylene manufacturers strive for a high ATE for ethylbenzene, combined with a low-loss of $C_8$-aromatics and a high selectivity to the most desired xylenes.

It is an object of the invention to find a catalyst to be used in a process for the isomerization of $C_8$-alkyl aromatics, whereby a high ATE for ethylbenzene is obtained and furthermore it is an object to have minimal losses of $C_8$-aromatics.

US-A-3,767,721 discloses a catalyst composition for isomerizing alkyl benzenes, comprising (a) alumina - supported platinum and (b) a partially dealkalized mordenite form zeolite. Both parts of this catalyst composition are prepared separately, and subsequently they are mixed and moulded into pellets.

Surprisingly there has now been found a catalyst composition, suitable for use in a process for the isomerization of $C_8$-alkyl aromatics, comprising a Group VIII metal and an alkali metal containing zeolite and a binder material as support, the amount of alkali metal being between 2 and 3% by weight on the zeolite, which is preparable by a process, wherein an alkali containing zeolite is prepared and extruded together with a binder material, the resulting extrudate is calcined, loaded with a compound of a Group VIII metal and further calcined, followed by reducing the Group VIII metal.

Group VIII metals of the Periodic System of Elements are, nickel, iron, cobalt, platinum, palladium, osmium, iridium, ruthenium and rhodium. Platinum is the preferred noble metal. The amount of metal may vary within wide limits. In general the amount ranges from 0.1 to 5% by weight, calculated on the weight of the total carrier. In the case of platinum the amounts preferably range from 0.1 to 1% by weight, calculated on the weight of total carrier, preferably from 0.2 to 0.8, more preferably from 0.3 to 0.5% by weight, calculated on the weight of total carrier. The weight of the total carrier is the weight of the zeolite and the binder material.

The zeolites used in the process according to the invention are preferably mordenites.

Mordenites are crystalline natural or synthetic zeolites of the alumino-silicate type; they generally have a

2

composition, $Na_8Al_8Si_{40}O_{96}.xH_2O$ (x typically being around 24).

Instead of all or part of the sodium, other alkali metals may be present.

The resultant mordenite usually has the form of a crystalline powder.

To prepare H-mordenite, the cations in natural or synthetic mordenite can be replaced by hydrogen ions by reacting the mordenite, for example, with an aqueous solution of an acid as a result of which cations are exchanged for hydrogen ions, or by treatment, for example, with an aqueous solution of an ammonium salt, as a result of which cations are exchanged for ammonium ions and $NH_4$-mordenite is obtained. Subsequent calcination decomposes the ammonium ions into protons bound to the mordenite and ammonia. Ion-exchange may also take place after shaping e.g. extrusion of the mixture of mordenite and binder.

The amount of alkali metal in the mordenite is between 2 and 3% by weight on the mordenite. If, for example, a desired sodium level of 2 to 3% must be obtained, the mordenite may be treated with an aqueous solution of ammonium chloride.

The zeolite is used in combination with a binder material as support. Binder materials are for example, natural clays, such as kaolin and bentonite, and inorganic oxide, such as alumina, silica, boria or zirconia or combinations thereof. Preferred binder materials are aluminas such as pseudo-boehmite or gamma alumina.

The amount of zeolite and binder material may vary within wide ranges. A preferred composition comprises an amount of mordenite in the range of from 10 to 55, more preferred from 10 to 35% by weight, based on the weight of the total composition.

In the catalyst composition the alkali metal ion for example sodium, is present as a counter ion for the anionic framework of the zeolite. The platinum can be partially sulphided and located on the zeolite or on the binder or on both.

The noble metals can be supported on the catalyst by known techniques, such as impregnation, percolation, ion-exchange, for example competitive ion-exchange, with generally aqueous solutions of compounds of one or more of the noble metals. In a preferred process the zeolites are extruded together with the binder material, the resulting extrudate is calcined and loaded with a compound of a Group VIII metal and further calcined, followed by reducing the metal. The alkali metal containing zeolite was prepared by adjustment of the alkali level by ion-exchange within a pre-selected range. The starting mordenite material, which is extruded together with the binder material contains an alkali content between 2 and 3% by weight (on the weight of the mordenite) preferably an alkali content between 2.2 and 2.6% by weight.

The catalyst may be present in the shape of pellets, tablets, spheres, pills, saddles, trilobes, tetralobes, hollow cylinders, doughnuts and briquettes.

The catalyst composition according to the invention is useful in a process for the isomerization of $C_8$-alkyl aromatics. A hydrocarbon stream comprising a non-equilibrium mixture of $C_8$-alkyl aromatics is converted into a near-equilibrium mixture.

In a feed-stream from the refinery, the percentage of ortho-xylene and para-xylene may be augmented by the use of a catalyst composition according to the invention. The feed stream comprises ethylbenzene, and o-, m- and p-xylene. When ethylbenzene is not present in the feed-stream, the isomerization of the xylenes may be carried out without platinum, since then a hydrogenation/dehydrogenation function is not necessary. Without being bound to any theory it can be said that the acid sites on the zeolite suffice to isomerize the xylenes. When ethylbenzene is present in the feed and it must be converted into a particular xylene or xylenes, than a hydrogenation/dehydrogenation function, like a noble metal for example, platinum is necessary. It is in the latter reaction that $H_2$ is necessary. According to investigations the platinum and $H_2$ are necessary to cause the partial hydrogenation of the aromatic ring of ethylbenzene. Via rearrangement to a five-membered ring and again the formation of a six-membered ring and dehydrogenation xylenes are formed. For the sake of convenience the whole process is called isomerization, which strictly spoken is only applicable where real isomerization takes place. Isomerization is carried out at elevated temperature and pressure. Temperatures may vary from 360 °C to 500 °C, preferably from, 390 °C to 460 °C. Hydrogen pressure may vary from 5 to 80 bar, preferably from 8 to 40 bar (from 500 to 8000 kPa, preferably from 800 to 4000 kPa).

The hydrogen is generally present in excess; suitable molar ratios of hydrogen to feed are between 1:1 and 30:1, preferably between 2:1 and 10:1. The isomerization reaction may be carried out in batch, semi-continuously or continuously.

<u>EXAMPLE 1</u>

<u>Preparation of the xylene isomerization catalyst.</u>

For the preparation of the xylene isomerization catalyst, mordenite powder with an initial sodium content of 5.65% by weight was used. The loss on ignition (LOI) of this material was 16% by weight.

At room temperature 179 g of the mordenite powder was added to 1600 ml of a 0.15 molar ammoniumchloride containing aqueous solution and stirred continuously for 1 hour in a 2 litre beaker equipped with a magnetic stirrer. Subsequently the slurry was filtered in a Buchner filter unit, and the filtercake was washed with 800 ml demineralized water.

After drying in a ventilated oven at 120 °C for 18 hours the sodium content of the mordenite was 2.22% by weight.

A mixture of 43.4 g of the exchanged mordenite powder (LOI = 7.8% by weight) and 221 g mixture a pseudoboehmite Catapal B was transferred to a mixmuller. This mixture of 20% by weight mordenite and 80% by weight Catapal B (dry base) was mixed thoroughly for 10 minutes prior to adding 2 g acetic acid and 130 g water. After additional mixing for 10 minutes, the mix was extruded on a small single-screw laboratory extruder (type Bonnot) with a 1.8 mm delrin dieplate.

The LOI of the total mix was 49.5% by weight and after extrusion, drying at 120 °C overnight and calcination at 500 °C for 2 hours, were yielded extrudates with a diameter of 1.5 mm and an average length of 5 mm.

The water pore volume of the extrudates was determined to be 0.53 ml/g. On the basis of this pore volume determination 50 g of the extrudates were impregnated with an aqueous solution consisting of 0.665 g $H_2PtCl_6$ (containing 30.4% by weight Pt), 1.335 g $HNO_3$ (100%) and 23 g water.

The impregnation was carried out in a rotating baffled roundbottom flask. The platinum containing aqueous solution was sprayed over the extrudates in 13 minutes by pumping it through a pneumatic nozzle with air as the dispersing gas. After impregnation the extrudates were equilibrated in the rotating flask for 1 hour. The impregnated extrudates were dried in a ventilated oven at 120 °C for 18 hours. The calcination of the dried extrudates was carried out in the same ventilated oven at 500 °C for 1 hour.

## Isomerization of $C_8$-alkyl aromatics

For the determination of the catalyst activity a performance test was carried out in a micro flow testing unit. The amount of catalyst used in such a test is 4 g. Before testing, the catalyst was reduced with hydrogen at 400 °C at a pressure of 12.5 bar and a hydrogen flow of 7.5 Nl/g/h. Subsequently the catalyst sample was deactivated at 450 °C with a mixture consisting of 80% by weight ethylbenzene and 20% by weight meta-xylene. The deactivation conditions were: WHSV 5 g/g/h, hydrogen/hydrocarbon molar ratio 8, pressure 12.5 bar. Total deactivation took 24 hours and was followed by the actual testing of the activity and selectivity at a temperature of 425 °C. A performance was carried out with a mixture of 70% by weight meta-xylene and 30% by weight ethylbenzene at a system pressure of 12.5 bar, a hydrogen/hydrocarbon molar ratio of 8, and at a space velocity of 2 h$^{-1}$. During the test the reaction product was analyzed using gas chromatography. Performance data were calculated from the analyses. The ATE-value for ethylbenzene was 74 and the loss of $C_8$-alkyl aromatics was 16%.

## EXAMPLES 2-4 and Comparative Examples A and B

A number of xylene isomerization catalysts were prepared which contained a different sodium content of the mordenite. The catalysts were all prepared in the same manner as described in Example 1.

The respective ATE-values for ethylbenzene and the loss of $C_8$-alkyl aromatics for the different catalysts are given.

| % Na in mordenite | ATE (ethylbenzene) | loss of $C_8$-alkyl aromatics |
|---|---|---|
| 2 2.60 | 85 | 25 |
| 3 2.56 | 65 | 9.5 |
| 4 2.53 | 77 | 19 |
| (1) 2.22 | 74 | 16 |
| A 4.46 | 32 | 3.5 |
| B 0.0015 | 80 | 54 |

## Claims

1. Catalyst composition, suitable for use in a process for the isomerization of $C_8$-alkyl aromatics comprising a Group VIII metal and an alkali metal containing zeolite and a binder material as support, the amount of alkali metal being between 2 and 3% by weight on the zeolite, which is preparable by a process, wherein an alkali containing zeolite is prepared and extruded together with a binder material, the resulting extrudate is calcined, loaded with a compound of a Group VIII metal and further calcined, followed by reducing the Group VIII metal.

2. Catalyst composition as claimed in claim 1, wherein the alkali methal containing zeolite is prepared by adjusting the alkali level by ion-exchange within a pre-selected range.

3. Catalyst composition as claimed in claim 1 or 2 wherein the zeolite is a modernite.

4. Catalyst composition as claimed in claim 3, wherein the mordenite is a partially exchanged H-mordenite.

5. Catalyst composition as claimed in claim 3, wherein the starting zeolite material is a partially ion-exchanged sodium-mordenite with an alkali content between 2.2 and 2.6% by weight.

6. Catalyst composition as claimed in one of the claims 3-5, wherein the amount of mordenite is in the range of from 10 to 35% by weight, based on the weight of the total composition.

7. Catalyst composition as claimed in one of the claims 1-6, wherein the Group VIII metal is platinium.

8. Catalyst composition as claimed in claim 7, wherein the amount of platinum is in the range of from 0.3 to 0.5% by weight, based on the weight of the total composition.

9. Catalyst composition as claimed in one of the claim 1-8, wherein the alkali metal is sodium, lithium, potassium, cessium or rubidium.

10. Catalyst composition as claimed in one of the claims 1-9, wherein the alkali metal is present in the form of a counter-ion for the anionic framework of the zeolite.

11. Catalyst composition as claimed in one of the claims 1-10, wherein the Group VIII metal is partially sulphided.

12. Catalyst composition as claimed in one of the claims 1-11, wherein the binder material is pseudo-boehmite or gamma-alumina.

13. Process for the isomerization of $C_8$-alkyl aromatics wherein the $C_8$-alkyl aromatics are contacted with a catalyst composition as claimed in any of claims 1-12.

**Patentansprüche**

1. Katalysatorzusammensetzung, geeignet für den Einsatz in einem Verfahren zum Isomerisieren von $C_8$-Alkylaromaten, umfassend ein Metall der Gruppe VIII und einen Alkalimetall, enthaltenden Zeolith und ein Bindemateral als Träger, wobei die Menge an Alkalimetall zwischen 2 und 3 Gewichtsprozent , bezogen auf den Zeolith, beträgt, die sich durch ein Verfahren herstellen läßt, in welchem ein Alkali enthaltender Zeolith hergestellt und zusammen mit einem Bindemateral extrudiert wird, worauf das resultierende Extrudat calciniert wird, mit einer Verbindung des Metalls der Gruppe VIII beladen wird und weiter calciniert wird, worauf das Metall der Gruppe VIII reduziert wird.

2. Katalysatorzusammensetzung wie in Anspruch 1 beansprucht, in welcher der das Alkalimetall enthaltende Zeolith durch Einstellen des Alkaligehalts in einem vorbestimmten Bereich mittels Ionenaustausch hergestellt wird.

3. Katalysatorzusammensetzung wie in Anspruch 1 oder 2 beansprucht, in welcher der Zeolith ein Mordenit ist.

4. Katalysatorzusammensetzung wie in Anspruch 3 beansprucht, in welcher der Mordenit ein teilweise ausgetauschter H-Mordenit ist.

5. Katalysatorzusammensetzung wie in Anspruch 3 beansprucht, in welcher das Ausgangs-Zeolithmaterial ein teilweise ionenausgetauschter Natriummordenit mit einem Alkaligehalt von 2,2 bis 2,6 Gewichtsprozent ist.

6. Katalysatorzusammensetzung , wie in einem der Ansprüche 3 bis 5 beansprucht, in welcher die Menge an Mordenit im Bereich von 10 bis 35 Gewichtsprozent, bezogen auf das Gewicht der Gesamtzusammensetzung, liegt.

7. Katalysatorzusammensetzung, wie in einem der Ansprüche 1 bis 6 beansprucht, in welcher das Metall der Gruppe VIII Platin ist.

8. Katalysatorzusammensetzung, wie in Anspruch 7 beansprucht, in welcher die Menge an Platin im Bereich von 0,3 bis 0,5 Gewichtsprozent, bezogen auf das Gewicht der Gesamtzusammensetzung, liegt.

9. Katalysatorzusammensetzung, wie in einem der Ansprüche 1 bis 8 beansprucht, in welcher das Alkalimetall Natrium, Lithium, Kalium, Cäsium oder Rubidium ist.

10. Katalysatorzusammensetzung, wie in einem der Ansprüche 1 bis 9 beansprucht, in welcher das Alkalimetall in der Form eines Gegenions für das anionische Gerüst des Zeoliths vorliegt.

11. Katalysatorzusammensetzung, wie in einem der Ansprüche 1 bis 10 beansprucht, in welcher das Metall der Gruppe VIII teilweise sulfidiert ist.

12. Katalysatorzusammensetzung, wie in einem der Ansprüche 1 bis 11 beansprucht, in welcher das Bindemateral Pseudo-Boehmit oder Gamma-Alumiumoxid ist.

13. Verfahren zur Isomerisierung von $C_8$-Alkylaromaten, in welchem die $C_8$-Alkylaromaten mit einer Katalsatorzusammensetzung, wie in einem der Ansprüche 1 bis 12 beansprucht, kontaktiert werden.

**Revendications**

1. Composition de catalyseur, appropriée pour être utilisée dans un procédé pour l'isomérization de composés aromatiques à alkylation en $C_8$, comportant un métal du groupe VIII et un métal alcalin renfermant une zéolite et un produit liant en tant que support, la quantité de métal alcalin se situant entre 2 et 3% en poids sur la zéolite, qui est susceptible d'être préparée par un procédé dans lequel une zéolite renfermant un agent alkalin est préparée puis extrudée conjointement avec un matériau liant, l'extrudat résultant est calciné, chargé avec un composé d'un métal du groupe VIII et calciné ultérieurement, suivi par une réduction du métal du groupe VIII.

2. Composition de catalyseur telle que revendiquée dans la revendication 1, dans laquelle la zéolite renfermant le métal alcalin est préparé en réglant le niveau de l'agent alcalin par échange d'ion dans une gamme pré-choisie.

3. Composition de catalyseur telle que revendiquée dans la revendication 1 ou 2, dans laquelle la zéolite est une mordénite.

4. Composition de catalyseur telle que revendiquée dans la revendication 3, dans laquelle la mordénite est une mordénite H partiellement échangée.

5. Composition de catalyseur telle que revendiquée dans la revendication 3, dans laquelle le produit de départ à base de zéolite est une mordénite sodique qui présente une teneur en élément alcalin entre 2,2 et 2,6% en poids.

6. Composition de catalyseur telle que revendiquée dans l'une des revendications 3 à 5, dans laquelle la quantité de mordénite se situe dans la gamme de 10 à 35% en poids, en se basant sur le poids de la composition totale.

7. Composition de catalyseur telle que revendiquée dans l'une des revendications 1 à 6, dans laquelle le métal du Groupe VIII est le platine.

8. Composition de catalyseur telle que revendiquée dans la revendication 7, dans laquelle la quantité de platine se situe dans la gamme de 0,3 à 0,5% en poids, en se basant sur le poids de la composition totale.

9. Composition de catalyseur telle que revendiquée dans l'une des revendications 1 à 8, dans laquelle le métal alcalin est du sodium, du lithium, du potassium, du césium ou du rubidium.

10. Composition de catalyseur telle que revendiquée dans l'une des revendications 1 à 9, dans laquelle le métal alcalin est présent sous la forme d'un contre-ion, pour le réseau anionique de la zéolite.

11. Composition de catalyseur telle que revendiquée dans l'une des revendications 1 à 10, dans laquelle le métal du Groupe VIII est partiellement sulfuré.

12. Composition de catalyseur telle que revendiquée dans l'une des revendications 1 à 11, dans laquelle le matériau liant est une pseudo-boehmite ou une gamma-alumine.

13. Procédé pour l'isomérisation de composés aromatiques à alkylation en $C_8$, dans lequel les composés aromatiques à alkylation en $C_8$ sont mis en contact avec une composition de catalyseur telle que revendiquée dans l'une quelconque des revendications 1 à 12.